(19) **Europäisches Patentamt**

**European Patent Office**

**Office européen des brevets**

(11) **EP 1 175 233 B1**

(12) **EUROPEAN PATENT SPECIFICATION**

(45) Date of publication and mention
of the grant of the patent:
**25.02.2004 Bulletin 2004/09**

(51) Int Cl.7: **A61L 27/02**, A61L 31/02

(21) Application number: **00925461.6**

(86) International application number:
**PCT/GB2000/001450**

(22) Date of filing: **27.04.2000**

(87) International publication number:
**WO 2000/066190 (09.11.2000 Gazette 2000/45)**

(54) **DERIVATIZED POROUS SILICON**

DERIVATISIERTES PORÖSES SILICIUM

SILICIUM POREUX DERIVE

(84) Designated Contracting States:
**DE ES FR GB IT**

(30) Priority: **01.05.1999 GB 9909996**
**10.03.2000 GB 0005707**

(43) Date of publication of application:
**30.01.2002 Bulletin 2002/05**

(73) Proprietor: **pSiMedica Limited**
**Worcestershire, WR14 3SZ (GB)**

(72) Inventors:
• **CANHAM, Leigh, Trevor**
**Malvern WR14 3PS (GB)**
• **BARRETT, Christopher, Paul**
**Malvern, Worcestershire WR14 3PS (GB)**

(74) Representative: **Bowdery, Anthony Oliver et al**
**QinetiQ Ltd**
**Cody Technology Park**
**A4 Bldg.,**
**Ively Road**
**Farnborough, Hampshire GU14 0LX (GB)**

(56) References cited:
**WO-A-97/06101         GB-A- 2 303 847**
**US-A- 5 763 399**

• **BURIAK J M ET AL: "LEWIS ACID MEDIATED FUNCTIONALIZATION OF POROUS SILICON WITH SUBSTITUTED ALKENES AND ALKYNES" JOURNAL OF THE AMERICAN CHEMICAL SOCIETY,US,AMERICAN CHEMICAL SOCIETY, WASHINGTON, DC, vol. 120, February 1998 (1998-02), pages 1339-1340, XP002920673 ISSN: 0002-7863**

• **BANSAL, ASHISH ET AL: "Alkylation of Si Surfaces Using a Two-Step Halogenation / Grignard Route" J. AM. CHEM. SOC. (1996), 118(30), 7225-7226 , XP002145038**
• **A JANSHOFF ET AL: "Macroporous p-type silicon Fabry-Perot layers. Fabrication, characterization and applications in biosensing" JOURNAL OF THE AMERICAN CHEMICAL SOCIETY,US,AMERICAN CHEMICAL SOCIETY, WASHINGTON, DC, vol. 120, no. 46, 25 November 1998 (1998-11-25), pages 12108-12116, XP002125399 ISSN: 0002-7863**
• **CANHAM, LEIGH T. ET AL: "Derivatized mesoporous silicon with dramatically improved stability in simulated human blood plasma" ADV. MATER. (WEINHEIM, GER.) (1999), 11(18), 1505-1507 , 17 December 1999 (1999-12-17), XP000902381**
• **BURIAK, JILLIAN M.: "Organometallic chemistry on silicon surfaces: formation of functional monolayers bound through Si-C bonds" CHEM. COMMUN. (CAMBRIDGE) (1999), (12), 1051-1060 , 1 June 1999 (1999-06-01), XP002145037**
• **BOUKHERROUB, R. ET AL: "New Synthetic Routes to Alkyl Monolayers on the Si(111) Surface" LANGMUIR (1999), 15(11), 3831-3835 , 21 June 1999 (1999-06-21), XP000925257**
• **DANCIL, KEIKI-PUA S. E.A: "A Porous Silicon Optical Biosensor : Detection of" J. AM. CHEM. SOC., vol. 121, no. 34, 1 September 1999 (1999-09-01), pages 7925-7930, XP002145036**
• **US 5763399**
• **Science, vol. 278, 1997, pp. 840-843**
• **GB2303847**
• **Thin Solid Films 297, 1997, ix-x**
• **Advanced Materials, vol. 8, no. 10, 1996, pp. 847-849**

EP 1 175 233 B1

- Advanced Materials, vol. 8, no. 10, 1996, pp. 850-852
- Advanced Materials, vol. 7, no. 12, 1995, pp. 1033-1037
- Thin Solid Films, vol. 297, 1997, pp. 304-307
- Advanced Materials, vol. 11, no. 3, Febr. 1999, pp. 265-267

**Description**

This invention relates to derivatized porous silicon, to biomaterial comprising derivatized porous silicon, and to applications of such biomaterial.

A biomaterial is here defined as a non-living material used in or on the surface of a living human or animal body. It is intended to interact with the biological environment into which it is introduced. Such biomaterials can be bio-inert, bioactive or resorbable, depending on their interaction with the living tissue of the human or animal body. A relatively bio-inert biomaterial, such as titanium, undergoes minimal corrosion and minimal fibrous encapsulation by the surrounding tissue. A bioactive biomaterial, such as Bioglass (RTM), undergoes corrosion and thereby encourages tissue growth on its surface. A resorbable biomaterial, such as a polylactide, undergoes sufficient continuous corrosion to be completely dissolved in the body over a period of time.

To varying extents, the practical viability of most biomedical devices and structures (i.e. devices and structures used in or on the surface of a living human or animal body) will depend upon such issues as stability of their constituent biomaterial and interactions between the biomaterial surface and the biological environment of the body within which or on which the device is placed. For some applications (e.g. reconstructive prosthetics, wound repair, biochip integration, drug delivery) biomaterial corrosion is desirable. The extent of the desired corrosion will depend on the specific application, but in many it is desirable that the biomaterial is substantially stable within its environment i.e. that corrosion takes place over a long period of time. For other applications (e.g. biosensing, biofiltration, neuro-interfacing) a stable interface between the biomaterial and its environment is needed, i.e. it is desirable that there is little or preferably no corrosion of the biomaterial. For biofiltration applications in particular, the biomaterial is also required to be porous, indeed often highly porous. The requirements of stability and porosity often conflict, as a material is made more porous its stability can often decrease.

The following prior art may be relevant to the present invention: Adv. Mater., vol 11, no 18, December 1999; Chem. Commun., vol. 12, June 1999; Langmuir, vol. 15, no. 11, June 1999; J. Am. Chem. Soc., vol. 121, no. 34, September 1999; J. Am. Chem. Soc. 120, 1998, pp 1339-1340; J. Am. Chem. Soc., vol.118, 1996, pp. 7225-7226; J. Am. Chem. Soc., vol. 120, 1998, pp 12108-12116; WO 97 06 101; Science, vol 278, 1997, pp. 840-843; Thin Solid Films 297, 1997, ix-x; Advanced Materials, vol. 8, no. 10, 1996, pp. 847-849; Advanced Materials, vol. 8, no.10, 1996, pp. 850-852, Advanced Materials, vol. 7, no. 12, 1995, pp. 1033-1037; Thin Solid Films, vol. 297, 1997, pp. 304-307; Advanced Materials, vol. 11, no. 3, February 1999, pp. 265-267, US 5,763,399; and GB 2,303,847.

Silicon has for many years not been considered a viable biomaterial due to its perceived bioincompatability. It has recently been shown that by introducing varying levels of porosity into silicon, its biocompatability can be increased. Porous silicon although biocompatable in some biological environments has not been found to be stable in living human or animal bodies or simulations thereof. Corrosion takes place in days or even hours. However, as stated above, there are many applications where stability or at least substantial stability of a biomaterial is desired.

According to a first aspect of the present invention there is provided derivatized porous silicon, having a substantially monomolecular layer, the monomolecular layer comprising one or more organic groups that are covalently bonded by hydrosilylation to at least part of the surface of the porous silicon, the structure and composition of the derivatized porous silicon being such that it is suitable for use as a biomaterial, the derivatized porous silicon being for use as a biomaterial, a biomaterial being a non-living material for use in or on the surface of a living human or animal body.

The derivatized porous silicon may be derivatized mesoporous silicon.

The derivatised porous silicon may have a composition and structure such that the corrosion rate of the derivatized mesoporous silicon material in (SHP) is a factor of at least two orders of magnitude lower than underivatized mesoporous silicon.

The porosity of the derivatized porous silicon may be at least 5%.

According to a second aspect of the present invention there is provided biomaterial comprising derivatized porous silicon.

According to a third aspect of the present invention there is provided a biomedical device comprising derivatized porous silicon.

For the absence of doubt, derivatized porous silicon is to be taken as porous silicon having a substantially monomolecular layer that is covalently bonded to at least part of its surface. The surface of the porous silicon. includes the surfaces of the pores. As is well known porous silicon is silicon that has been porosified, by anodization, stain etching, or photochemical etching in HF based solutions. Porous silicon fabricated in this way has a porosity greater than 0.1 % and more typically greater than 1%.

Derivatization of the porous silicon has been found to increase its stability.

According to a fourth aspect of the present invention there is provided a biofiltration device, which is adapted for operation in or on the surface of a human or animal body, comprising one or more derivatised porous silicon filters, the or each derivatised porous silicon filter comprising derivatized porous silicon having a substantially monomolecular layer, the monomolecular layer comprising one or more organic groups that are covalently bonded by hydrosilylation to at least part of the surface of the porous silicon, the structure and composition of the derivatized porous silicon being such that it is suitable for use as a biomaterial, the derivatized porous silicon being for use as a biomaterial, a biomaterial being a non-living material for use in or on the surface of a living human or animal body.

The or each or some of the filters preferably act as molecular sieves. They preferably allow some molecules e.g. nutrients and waste products to pass through them, but prevent other molecules e.g. components of the immune system such as macrophages and immunoglobulin molecules from doing so. The pore size of the or each or some of the filters preferably determines the molecules which pass through them. The diameter of the pores of the or each or some of the filters may be in the range 15-50nm. The or each or some of the filters may have a thickness of a few μms. The porosity of the or each or some of the filters is preferably at least 5%, and could be 10% or 15% or higher.

The biofiltration device may form part of a multi-element device. The multi-element device may be adapted for operation in or on the surface of a human or animal body. The multi-element device may be a biosensor. The biosensor may be adapted for operation in or on the surface of a human or animal body. The biosensor may monitor one or more physiological functions of the body. The biosensor may monitor one or more aspects of one or more fluids of the body. The biosensor may monitor glucose levels, and/or lithium ion levels and/or potassium and/or alcohol levels within the body.

According to a fifth aspect of the present invention there is provided an immunoisolation device, which is adapted for operation in or on the surface of a human or animal body, comprising a biofiltration device, which is adapted for operation in or on the surface of a human or animal body, comprising one or more derivatised porous silicon filters, the or each derivatised porous silicon filter comprising derivatized porous silicon having a substantially monomolecular layer, the monomolecular layer comprising one or more organic groups that are covalently bonded by hydrosilylation to at least part of the surface of the porous silicon, the structure and composition of the derivatized porous silicon being such that it is suitable for use as a biomaterial, the derivatized porous silicon being for use as a biomaterial, a biomaterial being a non-living material for use in or on the surface of a living human or animal body, characterised in that the biofiltration device is constructed in such a manner that, when the immunosiolation device is in operation in or on the surface of a human or animal body, at least one of the derivatised porous silicon filters excludes at least some molecules of the immune system from the immunoisolation device.

The immunoisolation device may comprise a silicon capsule, of thickness preferably less than or equal to 500μm. The immunoisolation device, and preferably the capsule, may be provided with one or more derivatized porous silicon filters. The derivatized porous silicon may be derivatized mesoporous silicon. The or each or some of the filters preferably exclude at least some molecules of the immune system from the device. Such molecules may be, for example, macrophages and immunoglobulin molecules. The or each or some of the filters preferably allow non-immune system molecules into and out of the device. Such molecules may be, for example, nutrients and waste products. The pore size of the or each or some of the filters preferably determines the molecules which pass through them. The diameter of the pores of the or each or some of the filters is preferably in the range 15-50nm. The or each or some of the filters may be produced by anodisation of one or more parts of the capsule. The or each or some of the filters may have a thickness of a few μms. The porosity of the or each or some of the filters is preferably at least 5%, and could be 10% or 15% or higher.

Cells may be placed within the device, to isolate them from components of the immune system, and may be cultured on the inner surfaces of the or each or some of the derivatized porous silicon filters. Such cells may be insulin-secreting cells (Islets of Langerhans), baby hamster kidney cells releasing ciliary neuro-trophic factor for treatment of amyotrophic lateral sclerosis, bovine adrenal chromaffin cells for treatment of intractable pain. In this case, the pore size of the or each or some of the filters is preferably large enough to allow nutrients for the cells to diffuse into the device and waste products and insulin to diffuse out of the device, but have a distribution of size such as to exclude all cells and specific proteins of the immune system from the device.

According to a sixth aspect of the present invention there is provided a battery device, which is adapted for operation in or on the surface of a human or animal body, comprising a power source and a biofiltration device, which is adapted for operation in or on the surface of a human or animal body, comprising one or more derivatised porous silicon filters, the or each derivatised porous silicon filter comprising derivatized porous silicon having a substantially monomolecular layer, the monomolecular layer comprising one or more organic groups that are covalently bonded by hydrosilylation to at least part of the surface of the porous silicon, the structure and composition of the derivatized porous silicon being such that it is suitable for use as a biomaterial, the derivatized porous silicon being for use as a biomaterial, a biomaterial being a non-living material for use in or on the surface of a living human or animal body.

The power source may comprise one or more bioluminescent organisms which emit light. The or each or some of the organisms may be microorganisms genetically modified with green fluorescent protein (GFP). This preferably realises high quantum yields (greater than 50%) and electrical power high enough to drive CMOS transistors. The or each or some of the organisms may contain luciferase enzymes which generates 560 nm light in the presence of ATP, $Mg^{2+}$, oxygen and luciferin. Preferably, body fluids containing nutrients, such as glucose, provide continuous energy for the organisms. The battery device may comprise one or more photodetectors, such as p-n junctions or p-i-n junctions. These may convert the light generated by the or each or some of the organisms into electrical power. The or each or some of the photodetectors may be used in conjunction with one or more mirrors, to enhance the light collection efficiency.

The power source may be an electrochemical power source. This may comprise at least one pair of electrodes. Power may be generated by electron transfer to and from the electrodes. The or each pair of electrodes may comprise dissimilar metals, e.g. aluminium and silver. Such a source preferably generates at least 0.8V. The or each pair of electrodes may be provided with an enzyme attached to one of the electrodes. The enzyme may be glucose oxidase. Preferably glucose is supplied to the battery which reacts with the glucose oxidase to produce hydrogen peroxide, which in turn reacts with the other electrode resulting in a transfer of electrons between the electrodes. Such a source preferably generates at least 2V.

The battery device may comprise a silicon box. The battery device, and preferably the box, may be provided with one or more derivatized porous. silicon filters. The derivatized porous silicon may be derivatized mesoporous silicon. The or each or some of the filters preferably exclude substances detrimental to the power source from the battery device. Such substances may include molecules of the immune system, proteins and enzymes. The or each or some of the filters preferably allow substances beneficial to the power source into the battery device. Such substances may include nutrients such as glucose and water. and waste products. The or each or some of the filters preferably allow substances produced by the power source to exit the battery device. Such substances may include waste products. The pore size of the or each or some of the filters preferably determines the substances which pass through them. The diameter of the pores of the or each or some of the filters is preferably in the range 15-50nm. The or each or some of the filters may be produced by anodisation of one or more parts of the battery device, preferably the silicon box. The or each or some of the filters may have a thickness of a few μms. The porosity of the or each or some of the filters is preferably at least 5%, and could be 10% or 15% or higher.

The battery device may provide power to one or more devices. The devices may be adapted for use in or on the surface of a human or animal body, or in vitro. Electrical connections may be provided between the battery device and the or each device. The or each or some of the devices may be microfluidic drug delivery devices, biosensors, nerve stimulation devices, identification/tagging devices.

According to a seventh aspect of the present invention there is provided an optical device, which is adapted for operation in or on the surface of a human or animal body, comprising a multilayer mirror comprises a stack of alternating layers of derivatized porous silicon, having a first porosity and a first refractive index, and derivatized porous silicon having a second porosity and a second refractive index which is higher than the first refractive index, the derivatized porous silicon having a substantially monomolecular layer, the monomolecular layer comprising one or more organic groups that are covalently bonded by hydrosilylation to at least part of the surface of the porous silicon, the structure and composition of the derivatized porous silicon being such that it is suitable for use as a biomaterial, the derivatized porous silicon being for use as a biomaterial, a biomaterial being a non-living material for use in or on the surface of a living human or animal body.

Lasers, and optics in general, are increasingly being utilised in health care for both non-invasive/minimally-invasive diagnostics and therapeutic treatment. Well known examples include pulse oximetry for monitoring the level of blood oxygenation, endoscopic fluorescence imaging for cancer detection, photodynamic therapy (PDT), non-invasive spectroscopy approaches to glucose monitoring, etc. A significant issue with all optical diagnostic techniques is quantification/control of the path length that the light from the source being used has travelled in vivo prior to detection. A significant issue with techniques such as PDT is the minimisation of damage to healthy tissue surrounding the cancerous site being treated. Both problems arise from the inhomogeneous, highly scattering, optical properties of tissue.

The device may be adapted for use in conjunction with a source of light. The device preferably controls the path length of the light from the source. This may be achieved by strategic placement of the device within the body.

The optical device may comprise a high, preferably greater than 95%, reflectivity structure. The optical device may comprise a multilayer mirror. The multilayer mirror may consist of a stack of alternating layers of derivatized porous silicon material having a first porosity and a first refractive index, and derivatized porous silicon material having a second porosity and a second refractive index which is higher than the first refractive index. The porosity may be inversely proportional to the refractive index. The first porosity may have a value in the region of 40%, and the second porosity may have a value in the region of 90%. The first porosity may have a value in the region of 50%, and the second porosity may have a value in the region of 71%. The layers of silicon material preferably have a thickness in the region of a quarter of the wavelength of the light incident upon them. The thickness of the layers preferably lies in the region 50-1000nm. If the light incident on the layers is in the blue region of the visible spectrum, i.e. has a wavelength of approximately 400nm, the thickness of the layers is preferably in the region of 100nm. If the light is in the near infra red spectrum, i.e. has a wavelength of approximately 2μm, the layer thickness is preferably in the region of 500nm. When the light incident on the mirror is in the visible or near infrared spectrums, the refractive indices of the layers preferably lie in the region 1.3-3.5. The reflectivity of the mirror is preferably high (e.g. over 95%) over a single or a range of wavelengths corresponding to the wavelength or wavelengths of the light incident thereon. This is referred to as the stop band of the mirror. The wavelength position and width of the stop band is preferably controlled by the design of the mirror stack, by such characteristics as the porosities of the silicon material used, and the number and thickness of the layers. The central wavelength of the stop band (known as the Bragg wavelength, $\lambda_{Bragg}$) is given by:

$$m\,\lambda_{Bragg} = 2\,(d_1 n_1 + d_2 n_2)$$

where m is the order of the Bragg condition, d refers to layer thickness, n to refractive index, and subscripts 1 and 2 to the first and second refractive indices. The refractive indices of the layers may be chosen such that the stop band of the mirror lies in the region 700-1000nm. This is the spectral range where living tissue has an 'optical window'. Very high, preferably greater than 95%, levels of reflectivity are preferably achieved. Using derivatized porous silicon in such optical devices improves their stability in comparison to previously known devices, and provides a means to prolong their lifetime in vitro or in or on the surface of a living human or animal body. For example, underivatized porous silicon multilayer mirrors dissolve in a few days in simulated human plasma (SHP), whereas derivatized mirrors may be stable in SHP for periods of weeks or months. When used in a body, the optical device is preferably eventually degradable in the body. It does not then have to be surgically removed once no longer needed, and problems related to permanently implanted devices are avoided.

The optical device is preferably at least substantially hydrophobic. This limits wetting of the device by aqueous fluids e.g. body fluids, which would otherwise penetrate the device causing corrosion thereof especially from within. Any corrosion of the hydrophobic device is then dominated by surface attack.

The reflectivity of the mirror may depend on the number of layers in the mirror. However, the reflectivity does not generally increase linearly with the number of layers, but saturates i.e. reaches a maximum value after a certain number of layers, e.g. ten layers, called the saturation layers. Addition of further layers above this number does not significantly increase the reflectivity. The mirror may comprise a number of layers greater than the number of layers required for saturation of the reflectivity. Light incident on the mirror will interact with the saturation layers. Layers beneath these will be initially 'redundant' layers, and will not significantly contribute to the reflectivity of the mirror. When corrosion of the mirror is dominated by surface attack, as the layers thereof are corroded away the reflectivity of the mirror will at least initially not be significantly affected. This is because as a layer is removed by corrosion, a previously redundant layer becomes one of the saturation layers, maintaining the number of these layers. This continues until the number of layers falls beneath the number required for saturation, the reflectivity of the mirror will then start to decrease. By making the number of the redundant layers large in comparison to the number of layers required for saturation, the maximum reflectivity may be maintained until the mirror has virtually corroded away. If the rate of corrosion is known, the number of redundant layers may be chosen to ensure that the reflectivity of the mirror remains at a maximum throughout the period in which the mirror is required to operate. The duration of the mirror in vitro or in or on the surface of a living human or animal body prior to resorbtion may be tuned by the number of layers therein.

The optical device may be capable of bonding to bone, in vitro or in or on the surface of a living human or animal body. This may be due to bone-bonding ability of derivatized porous silicon. When used in a living body, the optical device may be placed on bone, preferably close to the skin. The optical device may be placed in a subcutaneous site. The optical device may be used with an endoscope. For invasive therapeutic applications, the optical device could form part of a larger optical cavity device or micro-optical bench.

According to a eighth aspect of the present invention there is provided a cardiovascular device comprising derivatized porous silicon.

The cardiovascular device may be adapted for operation in or on the surface of a living human or animal body, or in vitro. The device may come into direct and possibly prolonged contact with blood. In such a case, the derivatized porous silicon is preferably haemocompatibile, and the surface thereof is preferably adapted such that clotting and/or calcification thereon are avoided. Underivatized bulk silicon is known to be thrombogenic from studies of blood clotting time.

The derivatized porous silicon preferably has one or more organic groups attached to the surface thereof. The organic groups may comprise hydrophilic polymer groups e.g. polyethylene oxide, and/or hydrophobic polymer groups e.g. polyurethanes. The polymer groups may contain polar phospholipid groups. Such organic groups are known to confer better haemocompatibility than silicon oxide, the normal surface component of underivatized porous silicon in physiological conditions. The organic groups may also be chosen for their ability to bind substances, such as heparin, albumin, phosphorylcholine or other biological agents. The organic groups may also be chosen for their ability to promote host cell overgrowth, e.g. overgrowth of endothelial cells (the cells that line the internal surfaces of blood vessels). The derivatized porous silicon preferably has a high surface area/volume matrix in which anti-calcification agents may be embedded. Using derivatized porous silicon minimises corrosion known to be a factor in promoting calcification.

According to an ninth aspect of the present invention there is provided a microelectrode device, which is adapted for operation in or on the surface of a human or animal body, comprising a plurality of electrical connections and a microelectrode comprising derivatized porous silicon, having a substantially monomolecular layer, the monomolecular layer comprising one or more organic groups that are covalently bonded by hydrosilylation to at least part of the surface of the porous silicon, the structure and composition of the derivatized porous silicon being such that it is suitable for use as a biomaterial, the derivatized porous silicon being for use as a biomaterial, a biomaterial being a non-living material for use in or on the surface of a living human or animal body.

Commercial biomedical microelectrodes often use porous coatings to improve tissue integration and thereby lower interfacial impedance. Such porous coatings however need to remain conductive and have excellent corrosion resistance when under electrical bias. Underivatized porous silicon microelectrodes would undergo significant corrosion in most physiological conditions of pH greater than 7, e.g. soft tissue, bone, muscle and blood. The application of electrical bias to the electrodes, corresponding to a positive surface charge, would accelerate this degradation. The impedance would rise with time and the ac drift would also be unacceptable. Using derivatized porous silicon in the manufacture of microelectrode devices seeks to alleviate these problems.

According to an tenth aspect of the present invention there is provided a wound repair device comprising derivatized porous silicon.

The wound repair device may be adapted for operation in or on the surface of a living human or animal body, or in vitro. The wound repair device may comprise derivatized porous silicon microvelcro. Such a device is porous and yet at least substantially stable in vitro and in or on the surface of a living human or animal body. The device may be impregnated, for example with one or more bioactive agents such as antibiotics and/or silver.

According to a eleventh aspect of the present invention there is provided a radiotherapy device, which is adapted for operation in or on the surface of a human or animal body, comprising a pellet formed, at least partly, from a radioisotope and derivatized porous silicon, having a substantially monomolecular layer, the monomolecular layer comprising one or more organic groups that are covalently bonded by hydrosilylation to at least part of the surface of the porous silicon, the structure and composition of the derivatized porous silicon being such that it is suitable for use as a biomaterial, the derivatized porous silicon being for use as a biomaterial, a biomaterial being a non-living material for use in or on the surface of a living human or animal body.

Radiotherapy is an effective treatment of cancers. Glass microspheres have been developed for in-situ irradiation. The radioactive material is embedded in the glass, which must have very low corrosion rates in body fluids to ensure that there is minimal radiation dose to neighbouring organs. Using derivatized porous silicon for the manufacture of radiotherapy devices ensures good stability thereof in vitro or in or on the surface of a living human or animal body. Derivatized porous silicon may be micromachined into a variety of shapes, the device may be shaped to match the shape of a physiological site to which it is intended to attach, e.g. a bone tumour.

According to an eleventh aspect of the present invention there is provided a drug delivery device comprising derivatized porous silicon.

The drug delivery device may be adapted for operation in or on the surface of a living human or animal body. By using derivatized porous silicon the stability of the device is substantially improved over existing devices, and the payload of the drug is preferably improved. The device may be capable of very long-term delivery (i.e. many months to years). Derivatization preferably also provides a means of covalently binding a range of therapeutic elements and/or low molecular weight drug molecules to the internal surface of the derivatized porous silicon. The improved stability of the device preferably aids electrical control of drug delivery. The derivatized porous silicon may comprise one or more functional groups bonded to the surface thereof. These preferably protect the underlying silicon from corrosion. They may be eventually degradable e.g. resorbable in physiological conditions. They preferably degrade to non-toxic products. They may be resorbable polymers, which may degrade into $CO_2$ and water after prolonged hydrolysis.

The derivatized porous silicon is preferably derivatized by a technique that does not involve oxidation of the silicon. This technique may result in derivatized porous silicon having Si-R termination, where R is one or more functional groups attached to the silicon via Si-C bonds. Using such a technique has a number of advantages. The derivatized porous silicon is more stable than underivatized porous silicon. Termination of the silicon via Si-C bonds prevents oxidation of the silicon, i.e. formation of $Si-O_x$ bonds on the surface thereof. This maintains the semiconducting nature of the material, silicon oxide being an insulator.

The porous silicon is preferably derivatized by hydrosilylation, and more preferably by Lewis acid mediated hydrosilylation. The Lewis acid may be $EtAlCl_2$. The hydrosilylation preferably involves covalent modification of the surface of the porous silicon, preferably by hydrosilylation of alkynes and/or alkenes yielding vinyl and/or alkyl groups bound to the surface of the porous silicon.

Derivatization preferably improves the stability of the porous silicon under oxidising conditions. The derivatized porous silicon is preferably stable to boiling in aerated water for preferably at least two hours. Unmodified (i.e. underivatized) porous silicon undergoes substantial oxidation and degradation in boiling water after one hour. The derivatized porous silicon is preferably at least substantially stable to boiling in aerated basic solutions of aqueous KOH (pH 10) and solutions of 25% EtOH/75% aqueous KOH (pH 10) for one hour. Unmodified porous silicon dissolves rapidly under these conditions.

Porous silicon can be subdivided according to the nature of the porosity. Microporous silicon contains pores having a diameter less than 20Å; mesoporous silicon contains pores having a diameter in the range 20Å to 500Å; and macroporous silicon contains pores having a diameter greater than 500Å. The derivatized porous silicon may be derivatized mesoporous silicon.

The corrosion rate of the derivatized mesoporous silicon material in simulated human plasma is preferably a factor of at least two orders of magnitude lower than underivatized mesoporous silicon.

The porosity of the derivatized porous silicon is preferably at least 5% (i.e. its void fraction or percentage of air may be 5%), but could be as high as 60% or 70%, 80% or 90%. The stability of such high porosity material demonstrates that for the first time high porosity structures can be realised that are both (a) not heavily oxidised and hence semiconducting in nature and (b) relatively stable for physiological environments. In comparison, underivatized high porosity (75%) mesoporous silicon undergoes some degree of corrosion under physiological conditions of pH 7, and is resorbable in vitro and in vivo. Thin films (5-10μm thick) of such underivatized mesoporous silicon are found to dissolve in simulated human plasma after one day.

According to a twelfth aspect, the invention provides a corrosion analysis system comprising:

(a) a source of electromagnetic radiation;
(b) a detector of electromagnetic radiation;
(c) a processing means;

characterised in that, when in use, the source is arranged such that it is capable of irradiating at least one multi-layer porous silicon or derivatised porous silicon mirror, the detector is arranged such that it is capable of detecting radiation reflected from said at least one mirror, and the processor means is adapted such that it is capable of processing a signal generated by said detector to yield information relating to corrosion of the or each mirror.

For example the source and detector may form part of a spectrometer for determining the reflectance or transmittance of the mirror or mirrors. The corrosion may result from implantation of the mirror in an animal or human body.

The processor means may be adapted such that it is capable of processing a signal generated by said detector to yield the number of layers present in the or each mirror.

Corrosion may result in loss of the number of layers from which the mirror is formed. The processor means may be adapted to provide information relating to the number of layers that have been lost or to the number of surviving layers.

Alternatively the processor means may be adapted such that it is capable of processing a signal generated by said detector to yield the amount of any substance that has been eroded from the or each mirror.

The mirror may comprise a substance, such as a drug or a mineral. As the mirror is corroded the substance may be released into the body of the animal or human. The processor means may be adapted such that it is capable of yielding information relating to the amount of the substance that has been lost through corrosion, or information relating to the amount of the substance that survives in the uncorroded part of the mirror.

The corrosion analysis system may further comprise said at least one mirror.

Embodiments of the invention will now be described by way of example, with reference to the accompanying drawings, in which:

**Figure 1**  is a schematic representation of the derivatization of hydride terminated porous silicon through a Lewis acid mediated hydrosilylation reaction of 1 dodecyne;

**Figures 2 (a), (b), (c) and (d)**  show plan and cross sectional scanning electron microscopy (SEM) images of underivatized porous silicon (a, b) before SHP exposure, and derivatized porous silicon (c, d) after 4 weeks immersion in SHP;

**Figures 3 (a), (b) and (c)**  show plan view SEM images of underivatized porous silicon surface after varying times in SHP (a) 1 hour, (b) 5 hours, (c) 70 hours;

**Figures 4 (a), (b) and (c)**  show secondary ion mass spectroscopy (SIMS) depth profiles of the oxygen content of (a) derivatized porous silicon prior to SHP exposure but after 6 weeks aging i.e. storage in air, (b) underivatized porous silicon after 5 hours SHP exposure, and (c) derivatized porous silicon after 4 weeks SHP exposure;

**Figures 5 (a), (b) and (c)**      show Fourier transform infra red spectroscopy (FTIR) spectra of (a) freshly derivatized porous silicon, (b) derivatized porous silicon after 4 weeks in SHP, and (c) derivatized porous silicon after 2 months in ambient air;

**Figures 6 (a) and (b)**      show cross sectional and plan views of an immunoisolation device;

**Figure 7**      shows a cross sectional schematic view of a first embodiment of a battery device;

**Figure 8**      shows a cross sectional schematic view of a second embodiment of a battery device;

**Figure 9**      shows a schematic representation of a multilayer mirror;

**Figures 10 (a) and (b)**      show EDAx results for derivatised porous silicon mirrors;

**Figure 11**      shows the effect of incubation in SHP on an 80 layer mirror comprising dodecenyl terminated porous silicon;

**Figure 12**      shows the effect of incubation in SHP on a 40 layer mirror comprising dodecyl terminated oxidised porous silicon;

**Figures 13 (a) and (b)**      show reflectivity spectra for an 80 layer mirror comprising dodeceny terminated oxidised porous silicon before and after immersion in SHP;

**Figure 14**      shows a theoretical prediction of the variation of reflectivity with the number of layers of derivatised porous silicon;

**Figure 15**      shows a schematic diagram of a biofiltration device according to the invention;

**Figure 16**      shows a cardiovascular device according to the invention;

**Figure 17(a)**      shows a schematic diagram of a part of a wound repair device according to the invention;

**Figure 17(b)**      shows a schematic diagram of a microelectrode device according to the invention;

**Figure 18(a)**      shows a schematic diagram of a radiotherapy device according to the invention;

**Figure 18(b)**      shows a part of a drug delivery device according to the invention; and

**Figure 19**      shows a corrosion analysis system according to the invention.

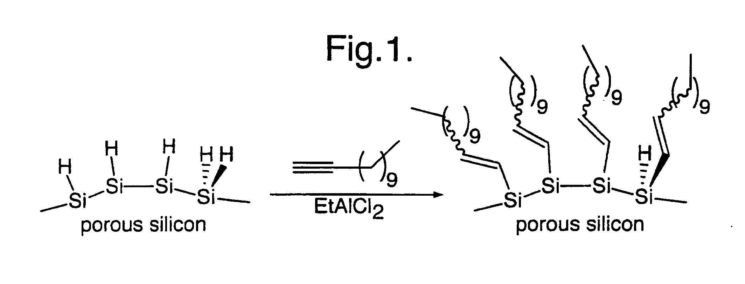

Figure 1 shows a schematic representation of the derivatization process on silicon wafers. These are (100) p-type boron doped wafers with resistivity of 7.5-8.5 $\Omega$cm. These were previously anodised galvanostatically at 1.7 mAcm$^{-2}$ in a 1:1 by volume mixture of 48% HF:$C_2H_5$OH for 5 minutes in the dark to yield a single layer of porous silicon. This single layer of porous silicon has a substantially uniform porosity throughout its thickness. Subsequent rinsing with ethanol and excess dry hexane was then carried out without permitting intermediate drying of the wafers. Derivatization was then carried out, using a Lewis acid (EtAlCl$_2$) mediated hydrosilylation to replace the silicon hydride termination of the wafers. Hydrosilylation was carried out with 1 dodecyne and yielded a dodecenyl terminated surface. The Lewis acid mediated hydrosilylation was performed in the following manner:

A hexane solution of the Lewis acid (EtAlCl$_2$) is bought into contact with the surface of the freshly anodized sample of porous silicon (comprising a single layer of uniform porosity). 1 dodecyne is then also placed on the surface of the porous silicon and the consequent reaction is allowed to proceed at an ambient temperature of 20 C for a period of 1 hour. The sample is then quenched with THF, followed by $CH_2Cl_2$. The whole process, from the application of the Lewis acid through to the quenching with $CH_2Cl_2$ is performed in an inert atmosphere. The derivatized sample is then rinsed in ethanol and dried under an $N_2$ stream.

The resulting surface is capped with a monolayer of dodecenyl groups. Such derivatized material only undergoes minor levels of oxidation even after one hour in boiling basic solutions (pH 10) of aqueous KOH. To put this into context, strongly basic solutions are frequently used to selectively dissolve many $\mu$m of porous silicon from wafers within seconds to minutes at room temperature.

The response of such wafers to physiological environments (pH 7.3) has been assessed. Derivatized material was exposed to SHP and its degree of corrosion, oxidation and calcification monitored by scanning electron microscopy (SEM), Fourier transform infra red spectroscopy (FTIR) and secondary ion mass spectroscopy (SIMS). These were compared with control wafers of the same microstructure, which were not derivatized and thus had hydride termination.

The derivatized and control wafers were incubated at 37° for periods of hours to weeks in the acellular SHP. The ion concentration of the SHP is as follows:

| ION | CONCENTRATION (mM) |
|---|---|
| $Na^+$ | 142.0 |
| $K^+$ | 5.0 |
| $Mg^{2+}$ | 1.5 |
| $Ca^{2+}$ | 2.5 |
| $HCO_3^-$ | 4.2 |
| $HPO_4^{2-}$ | 1.0 |
| $Cl^-$ | 147.8 |
| $SO_4^{2-}$ | 0.5 |

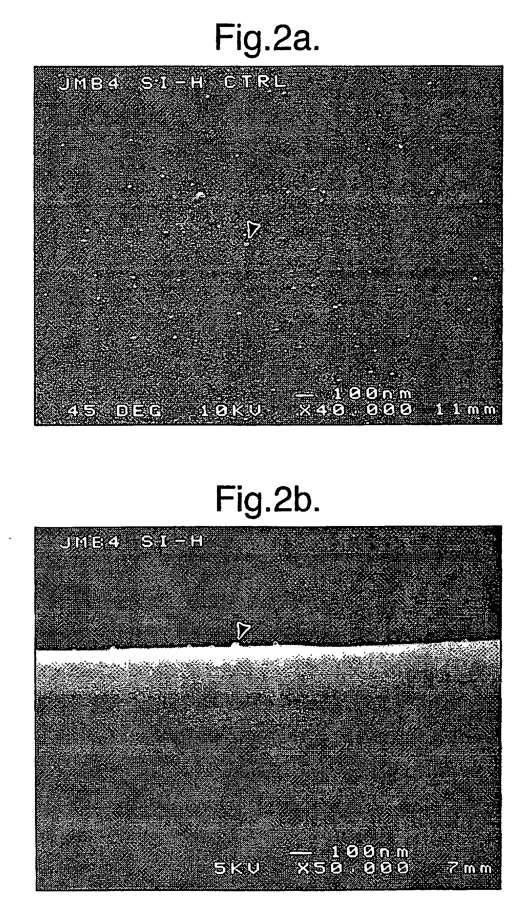

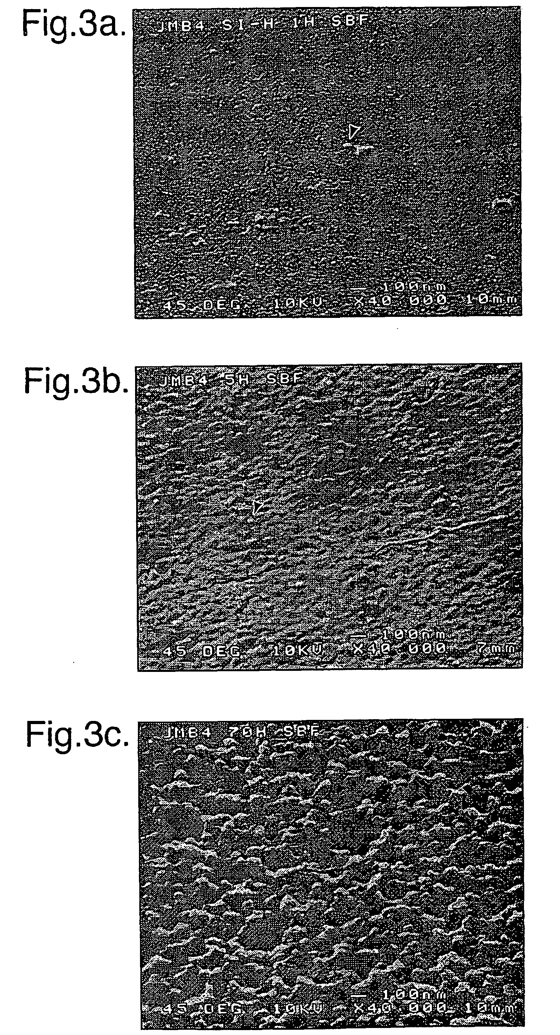

Figures 2(a) and 2(b) show the surface topography of a control wafer before SHP exposure. The porous silicon layer of the wafer is relatively thin ($275 \pm 15$nm at the centre of the 155mm$^2$ anodised area rising gradually to $350 \pm 15$nm at its circumference), and has some nanometre surface particulate contamination indicated by arrows. Figure 3(a) reveals the rapid increase in surface roughness of the control material that occurs within one hour exposure to this simulated physiological environment. After 5 hours (Figure 3(b)) there is evidence for a combined dissolution-deposition process occurring, and by 70 hours (Figure 3(c)) large areas of the control wafer had been completely removed, with that remaining having a heavily roughened appearance.

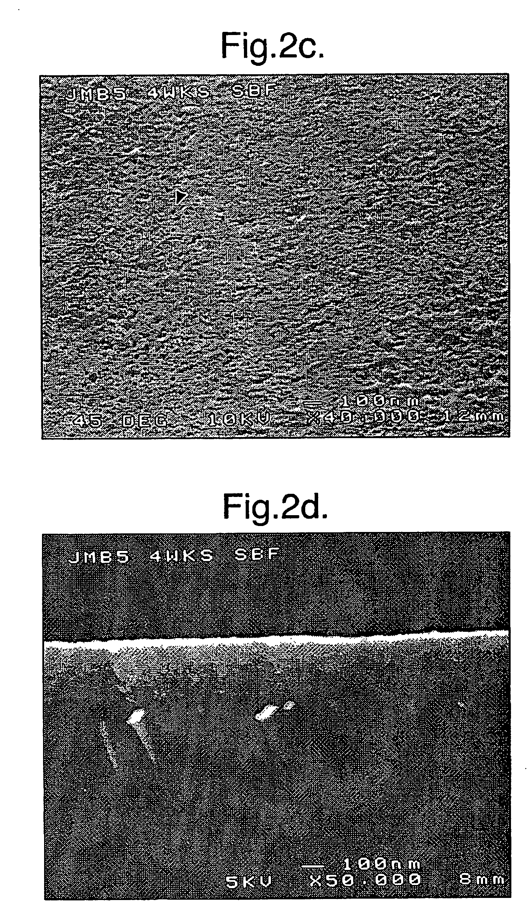

Figures 2(c) and 2(d) show the surface topography of a derivatized wafer after 4 weeks immersion in SHP. In striking contrast, the derivatized porous silicon layer thickness is essentially unchanged. Much of the change in surface topography of Figure 2(c) compared with that of Figure 2(a) is likely to arise from very thin SHP deposits. The nanometre scale pitting corrosion arrowed appears to correlate with surface particulates present after anodisation but prior to derivatization. Assuming they locally shield small areas from dodecenyl termination, which then become undercut, this form of corrosion is not intrinsic to the derivatization process nor derivatized material.

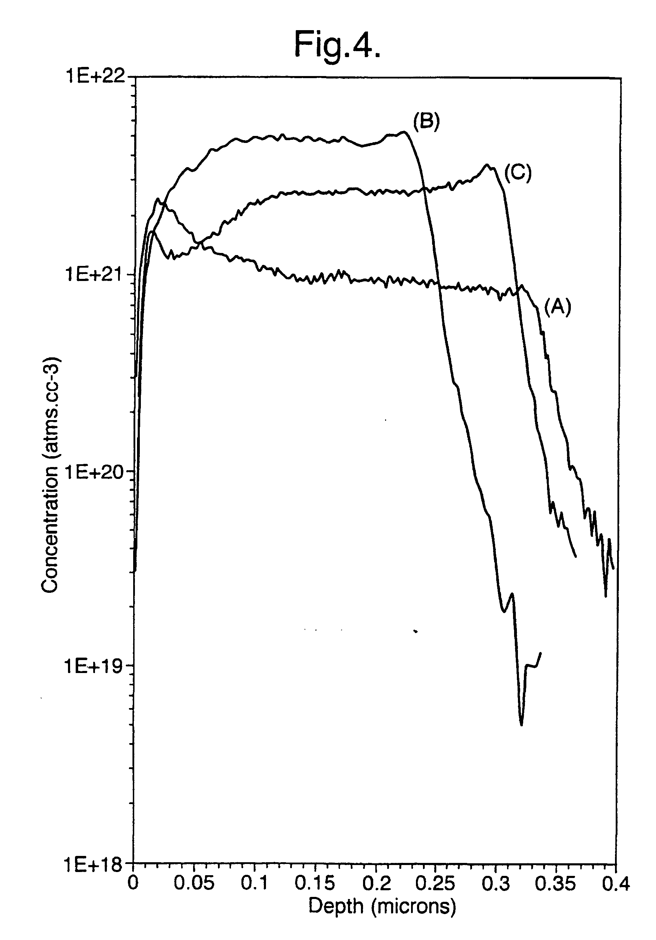

A comparison of Figures 2 and 3, with the additional observation that after 70 hours most of the 275nm thick underivatized porous silicon layer had been completely removed, indicates the dramatic change in stability brought about by this derivatization process. From Figures 2(a) and 2(d) and Figure 4 one can estimate that any layer thinning over the approximately 4 week (700 hour) period is $\leq$ 25nm for the derivatized material, but on average approximately 250 nm over 70 hours for the underivatized control material. Consequently the corrosion rate over these time periods and under these physiological conditions has been reduced by at least a factor of 100.

The extent to which the derivatized porous silicon has been infiltrated by the SHP and undergone oxidation has been investigated. SIMS profiles revealed substantial levels of Na, K, Cl Mg and Ca throughout the depth of the wafer. Since these elements are present in SHP but have very low levels in both freshly etched and aged (in ambient air) porous silicon, there is little doubt that the SHP solution has infiltrated the pores of the silicon to some degree. Figures 4(a), (b) and (c) compare the oxygen levels in aged derivatized porous silicon to that of SHP treated underivatized and derivatized porous silicon. SIMS analysis was conducted towards the circumference of the anodized area for each of the three materials indicated, where cross sectional SEM images indicated an initial wafer thickness of $315 \pm 15$ nm. The underivatized porous silicon has a higher degree of oxidation after 5 hours in SHP (and has been noticeably thinned) than the derivatized porous silicon after 4 weeks immersion. Nonetheless, it is clear that some additional oxidation of the derivatized porous silicon has occurred in SHP as compared with derivatized porous silicon stored in air for 6 weeks.

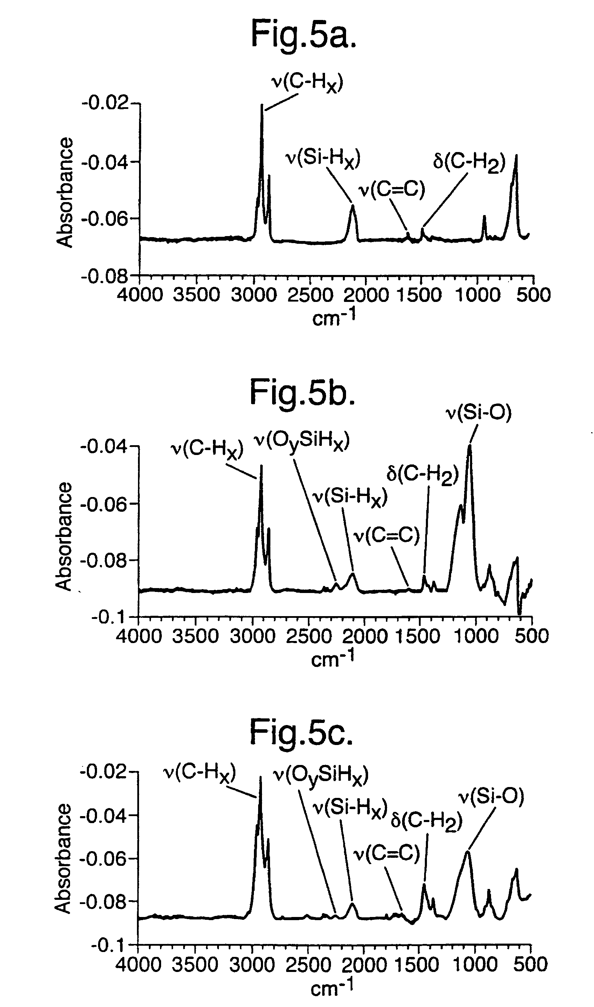

The above is verified by FTIR analysis (Figure 5). The relative amounts of silicon back-bonded to oxygen appear similar to the ambient air aged control material, but the Si-O stretch mode around 1100 cm$^{-1}$ in the SHP immersed material is significantly greater. This would be consistent with the backbone of the porous silicon undergoing hydrolysis, whilst its hydrophobic surface groups protect the surface, keeping it intact. The $\nu$ (c=c) stretch diminishes in intensity after 4 weeks immersion in SHP as can be observed upon comparison of Figures 5(a) and 5(b), possibly due to isomerization of the predominantly cis form of the double bond to the more thermodynamically stable trans confirmation under these conditions. In the case of the porous silicon material stored in air for 6 weeks, adsorption of hydrocarbon impurities takes place, as indicated by the change in ratio of $\nu$ (CH$_3$) and $\nu$ (CH$_2$) at 2690 cm$^{-1}$ and 2925 cm$^{-1}$ respectively, and by the increase in the intensity of $\delta$ (CH$_2$) at 1460 cm$^{-1}$.

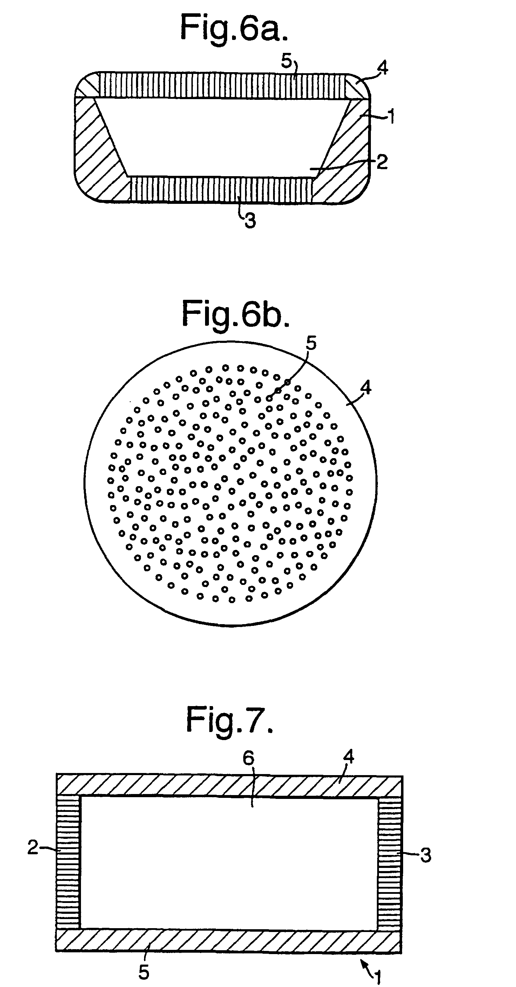

Figures 6 (a) and (b) show cross sectional and plan views of a immunoisolation device for containing insulin-secreting cells. This comprises a capsule of single crystal silicon wafer 1, having a reservoir 2 containing the insulin-secreting cells, a derivatized mesoporous silicon filter 3 and a lid 4 provided with a derivatized mesoporous silicon filter 5. The capsule is used in a living human or animal body, and the cells interface with the body via the filters.

The reservoir is photolithographically defined, by using an anisotropic etchant such as KOH. The capsule lid comprises a commercially available silicon membrane, and is bonded to the capsule using a very thin layer, e.g. less than 1μm, of medical adhesive known to be resistant to hydrolysis, such as cyanoacrylate or dental adhesive or silicone elastomer. Alternatively, a direct silicon to silicon bond or silicon to SiO$_x$ to silicon bond can be used, formed by a process which does not raise the temperature of the capsule by more than 30°C, so as not to damage the cells. The dimension of the capsule from filter 3 to filter 5 is 500μm or less. This ensures that the insulin secreting cells are not more than 500μm from blood vessels or other sources of nutrients, which would cause them to work poorly or even die. Thicker capsules can be realised, and have the advantage of being able to hold larger numbers of cells. However, the internal surfaces of such capsules have to be seeded with cells such as endothelial cells to help support the cells placed in the capsule. The derivatized porous silicon filters 3,5 are provided by anodisation of portions of the capsule and the lid. They have thicknesses of a few μms, and porosities in excess of 5% for 50nm diameter pores and 15% for 15-30nm diameter pores. This allows sufficient nutrient levels to reach the insulin-secreting cells, and have sufficient diffusional throughput to allow rapid insulin release in response to changing glucose levels in the body.

Figure 7 shows a cross sectional schematic view of a first embodiment of a battery. This comprises a substantially hollow silicon box 1 having first and second derivatized mesoporous silicon filters 2,3, and first and second photodetectors 4,5. The photodetectors are manufactured from silicon and comprise p-n junctions. A bioluminescent organism containing green fluorescent protein is contained within the cavity 6 of the box. Light produced by the organism is received by the photodetectors 4,5, and converted to electrical power. The filters 2,3 allow nutrients such as glucose to pass into the box and waste products to leave the box, but prevent components of the immune system, which might destroy the organism, from entering the box.

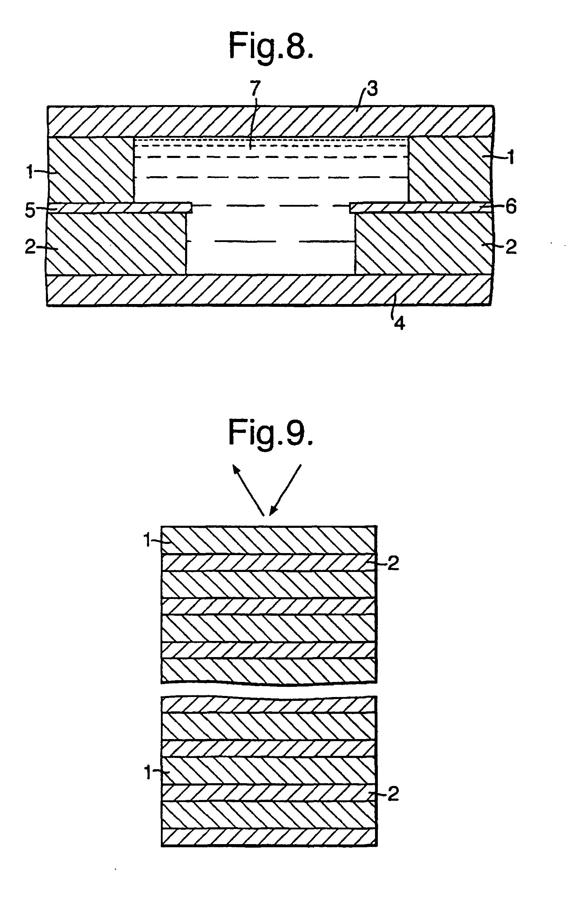

Figure 8 shows a cross sectional schematic view of a second embodiment of a battery. This comprises first and second layers of bulk non-porous silicon 1,2, and first and second derivatized porous silicon filters 3,4. First and second electrodes 5,6 are held between the layers of bulk silicon. The cavity 7 formed between the bulk and porous silicon contains a fluid, e.g. a body fluid. The first electrode 5 comprises aluminium, and the second electrode 6 comprises silver. Electron transfer occurs between the electrodes through the fluid, generating electrical power. This electrode system generates about 0.8V, and has a short circuit current determined by the electrode area. The electrodes are provided with electrical connections (not shown), to channel the power out of the battery. The filters 2,3 prevent substances detrimental to the electrodes from coming into contact them. In a further embodiment, the first electrode 5 has glucose oxidase enzyme anchored thereto. Glucose entering the battery via the filters is catalysed by the enzyme to yield hydrogen peroxide. This takes place in the following reaction at the second electrode 6:

$$H_2O_2 + 2H^+ + 2e^- \rightarrow 2H_2O$$

This results in electron transfer between the electrodes generating electrical power. This electrode system generates about 2V. The filters allow substances beneficial to the electrodes e.g. glucose to pass into the battery, but prevent substances detrimental to them from entering the battery.

Figure 9 is a schematic representation of a multilayer mirror. Two types of multilayer mirror were fabricated: a 40 layer mirror and an 80 layer mirror. The mirrors were fabricated by anodization of 0.01Ωcm resistivity p-type silicon wafer using 20% ethanoic HF acid. The current is modulated between 0.75A, for 4.5 second intervals, and 4.55A, for 2.55 second intervals. The modulation is repeated for 40 cycles to produce the 80 layer mirror, or for 20 cycles to produce the 40 layer mirror. The modulation of the current in this way results in the formation of alternate layers of high 1 and low 2 porosity porous silicon. The high porosity porous silicon layers 1 have a porosity of 71% and a thickness of 180nm; the low porosity porous silicon layers 2 have a porosity of 50% and a thickness of 90nm. The thickness of the layers may be varied by varying the duration of the high and low current intervals. The anodized wafers were native oxide passivated by storing them in ambient air for a period of two years.

The 40 and 80 layer mirrors were derivatised by two different methods. The first method is similar to that described earlier for the derivatisation of a single layer of porous silicon, namely the Lewis acid/dodecyne hydrosilylation. As with the earlier method, described in relation to figure 1, the Lewis acid (EtAlCl$_2$) is applied to the porous silicon surface of the mirror. The 1-dodecyne is then also applied to the surface to bring about the hydrosilylation. This method of derivatisation results in dodecenyl terminated porous silicon. In contrast with the earlier method, however, the porous silicon is pre-treated with HF to remove the oxide layer that is present as a result of the 2 year passivation process.

The second method of derivatisation involves immersion of the mirror in trichlorododecylsilane for 24 hours at room temperature to yield dodecyl terminated oxidised porous silicon. In contrast with the first method, the mirror is not pretreated with HF to remove the oxide layer resulting from the passivation process. The sample is rinsed in ethanol and dried under vacuum.

Both derivatised and underivatised 40 and 80 layer mirrors were incubated in simulated human plasma (SHP) at 37 C and pH 7.3. Mirrors were removed after periods ranging from a few hours to many months and the composition analysed using a JEOL 6400F scanning electron microscope. The electron microscopy results for the underivatised mirrors showed evidence of corrosion within a few hours of incubation, and 1 day's incubation was sufficient to cause mirror disintegration upon air drying.

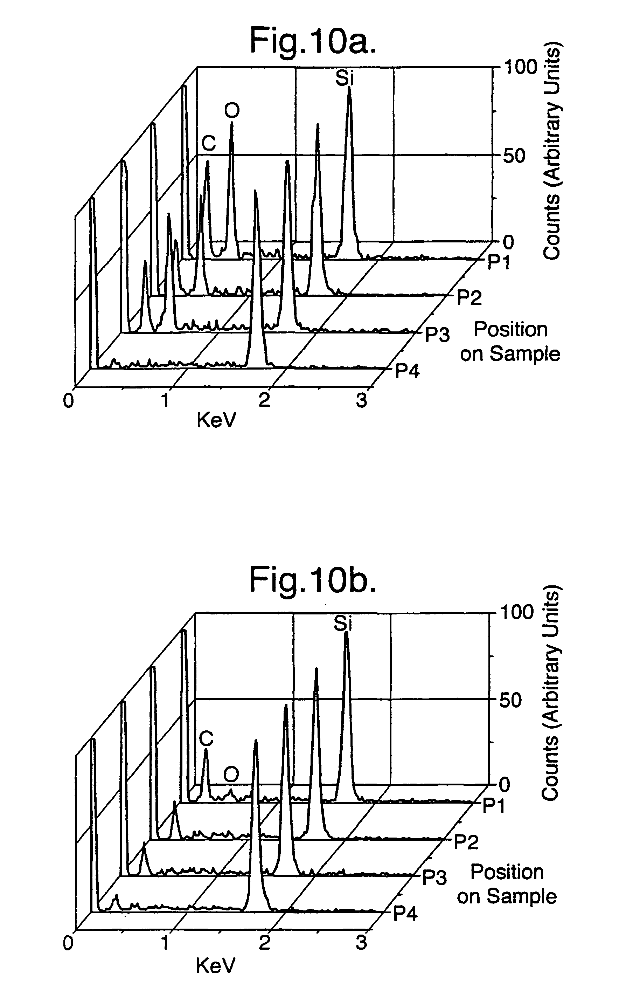

Derivatisation of the mirrors by either the first or second method was found not to introduce drying induced cracking or significant porosity gradients. EDAX results shown in figure 10 demonstrate impregnation of carbon through the full depth of the mirrors, showing that the pores of the mirrors do not become blocked during the derivatisation process. Figure 10a shows EDAX results for a porous silicon mirror derivatised by the second method. Figure 10b shows EDAX results for a porous silicon mirror derivatised by the first method.

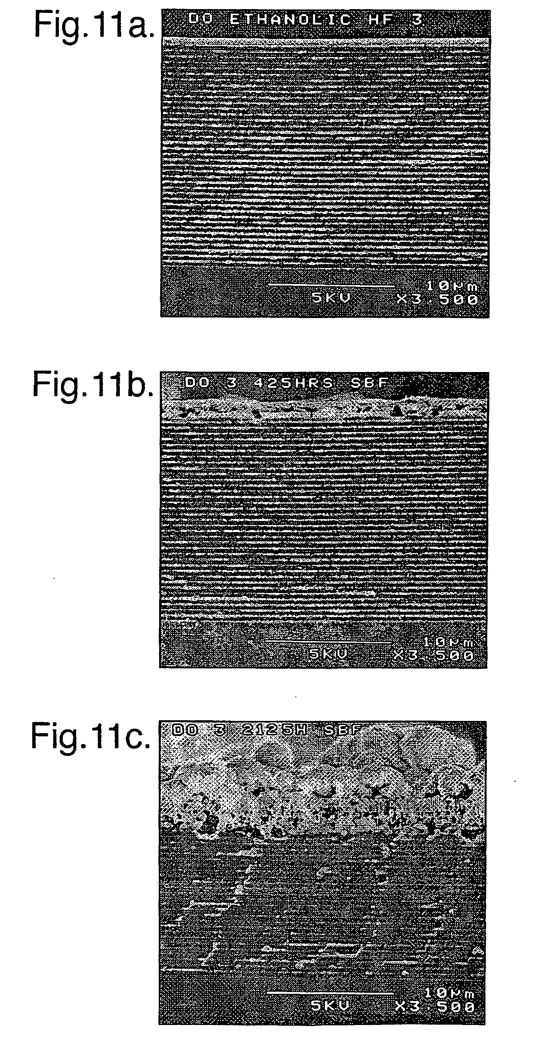

Figure 11 shows the effect of incubation in SHP on an 80 layer mirror comprising dodecenyl derivatised porous silicon. Figure 11a shows the mirror prior to incubation, figure 11b shows the mirror after 425 hours of incubation, and figure 11c shows the mirror after 2125 hours of incubation. After 425 hours 72 of the original 80 layers remain intact, after 2125 hours approximately 50 layers remain intact beneath the deposits of hydroxyapatite. This eventual calcification has slowed down the rate of dissolution; it would take more than 6 months for the the derivatised porous silicon layers to be completely dissolved.

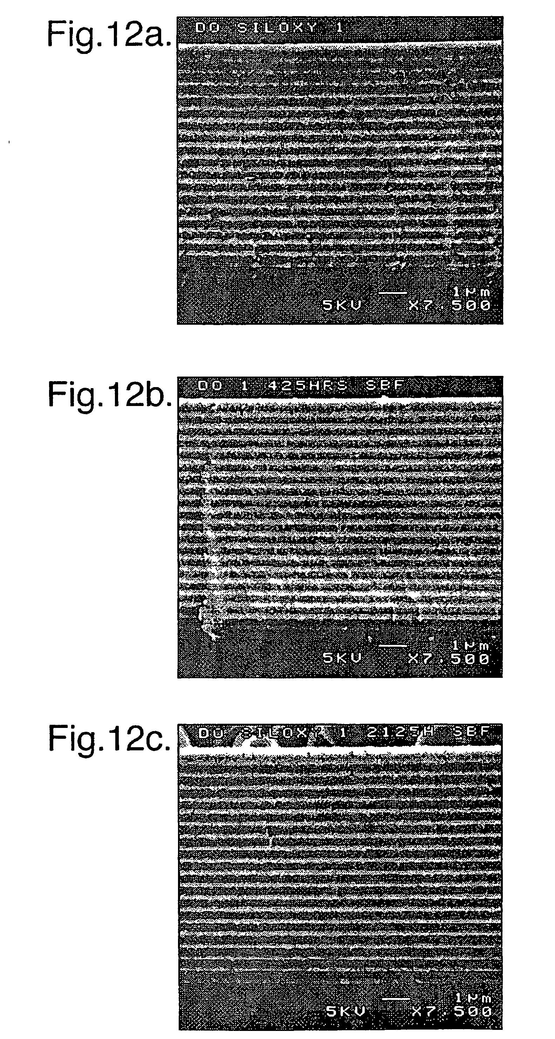

Figure 12 shows the effect of incubation in SHP on a 40 layer mirror comprising dodecyl derivatised porous silicon. Figure 12a shows the 40 layer mirror prior to incubation, figure 12b shows the 40 layer mirror after 425 hours of incubation, and figure 12c shows the mirror after 2125 hours of incubation. After 2125 hours the topmost layer is heavily oxidised, but has not dissolved. If a linear corrosion rate is assumed, complete dissolution would take approximately 10 years.

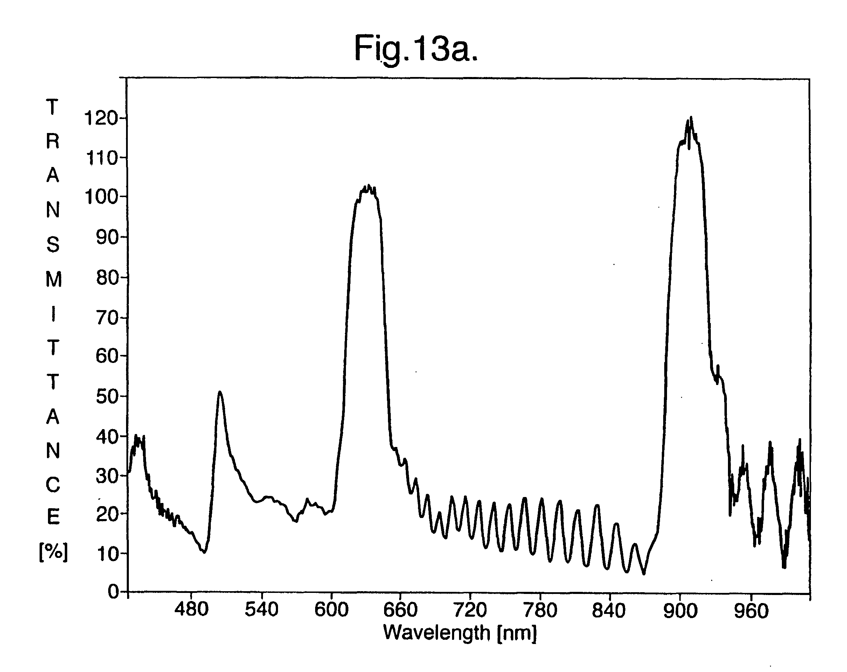

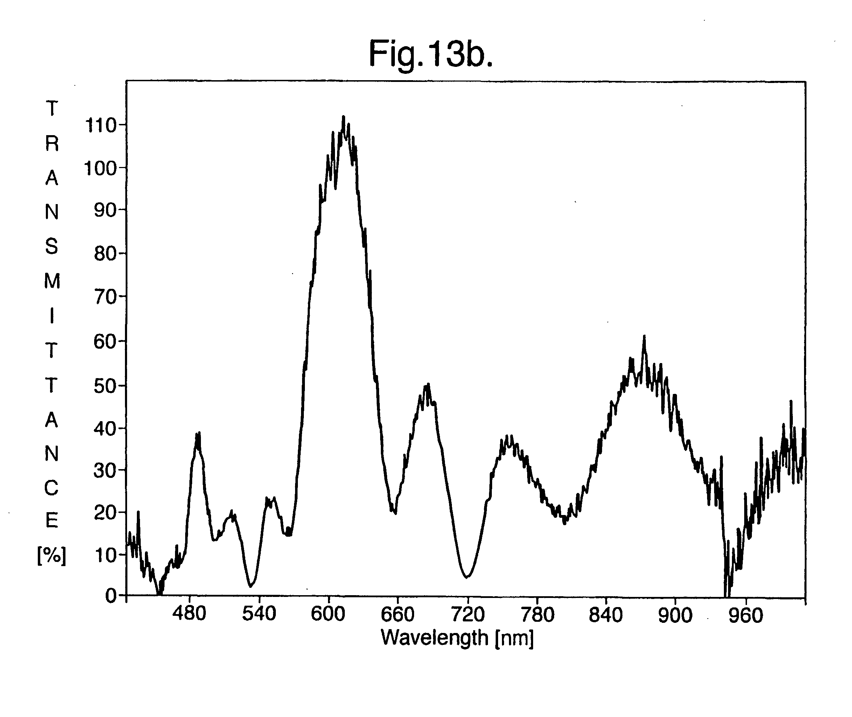

Figures 13a and 13b show reflectivity spectra for a 40 layer mirror comprising dodecenyl terminated porous silicon before and after immersion in SHP. Figure 13a shows the reflectivity before immersion and figure 13b shows reflectivity after immersion for 2125 hours. These results show that corroded structures continue to function as mirrors.

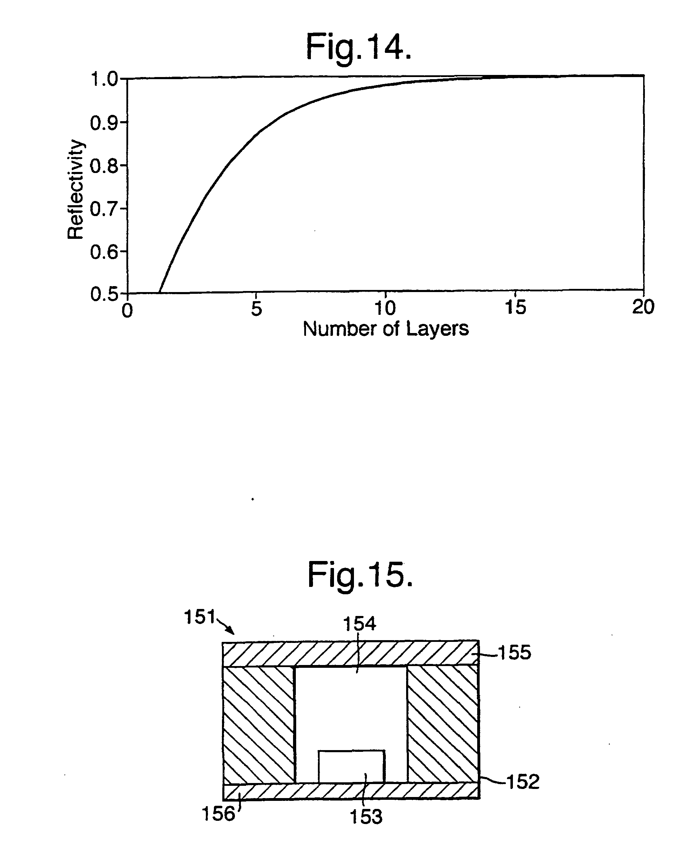

Figure 14 shows a theoretical prediction of the variation of reflectivity with the number of layers of derivatised porous silicon. The prediction shows that even if only a relatively small number of layers remain, reflectivity remains high.

Figure 15 shows a schematic diagram of a biofiltration device, generally indicated by 151, according to the invention. The device 151 includes a housing 152, a glucose sensor 153, a cavity 154, a derivatized porous silicon filter 155, and a cavity closure wall 156. The biofiltration device 151 is fabricated by etching a silicon wafer to form the cavity 154 and then porosifying the surface opposite to that of the cavity. The porous silicon is then derivatised, the sensor 153 is bonded to the closure wall 156, which is in turn bonded to the housing 152 so that the sensor is disposed in the cavity 154. Medical adhesive is used for bonding the sensor 153 to the closure wall 156 and the closure wall 156 to the housing 152.

The device 151 may be located in the blood stream or tissue of a patient. The filter 155 allows glucose molecules to pass through, while preventing blood cells and other material from reaching glucose sensor 153. The use of derivatized porous silicon is advantageous because it reduces deposition of material on the filter 155. In this way deposition on both the sensor 153 and filter 155 are minimised.

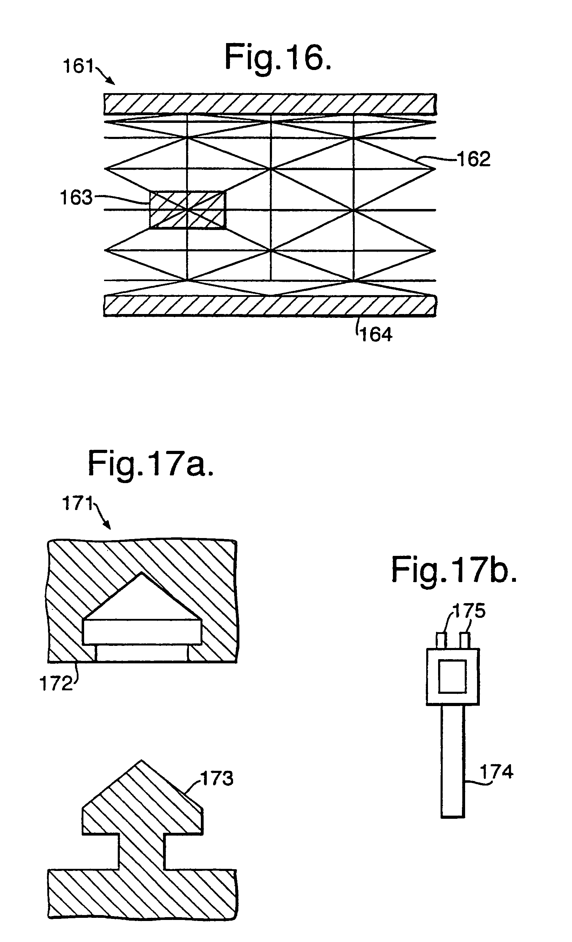

Figure 16 shows a schematic diagram of a cardiovascular device according to the invention. The cardiovascular device shown is a stent, generally indicated by 161, comprising a support scaffold 162 and a blood flow sensor 163. The stent may be used to support an artery wall 164, maintaining its diameter; the blood flow sensor 163 detecting the blood flow rate. The sensor 163 has an outer surface comprising derivatized porous silicon. The derivatisation may be selected such that clotting and/or calcification is minimised.

The sensor 163 allows the blood flow to be monitored; if an inappropriate blood flow is detected, then drugs are administered or the patient is operated upon to correct the situation. Sensors for the monitoring of blood flow or blood pressure, comprising derivatized porous silicon, may also be used in connection with other cardiovascular devices such as catheters.

Figure 17a shows a schematic diagram of part of a wound repair device according to the invention. The repair device comprises microvelcro, part of which is indicated by 171, that has an array of sockets 172 and plugs 173. The plugs 173 are formed from a first silicon wafer and the sockets from a second silicon wafer. The side of each silicon wafer, opposite to that of the plugs 173 or sockets 172, is attached to the tissue to be repaired. The two wafers are then drawn together so that the plugs 173 are secured in the sockets 172. The derivatization of porous silicon in this way allows the corrosion rate of the porous silicon to be controlled and reduces calcification. The use of a porous

EP 1 175 233 B1

material allows tissue to grow into the pores, facilitating the repair of the wound.

Figure 17b shows a schematic diagram of a microelectrode device, generally indicated by 171, according to the invention. The device includes a microelectrode 174, comprising derivatized porous silicon, and electrical connections 175; it may be used to electrically stimulate a body part or to monitor electrical activity within a patient. A control system (not shown), may be located at a distance from the point of electrical stimulation because of its relative bulk, and be connected to the microelectrode 174 by the electrical connections 175. The porous nature of the microelectrode 174 facilitates tissue integration thereby lowering interfacial impedence. The derivatization reduces corrosion of the porous silicon, so that the electrical properties of the electrode 174 remain relatively constant.

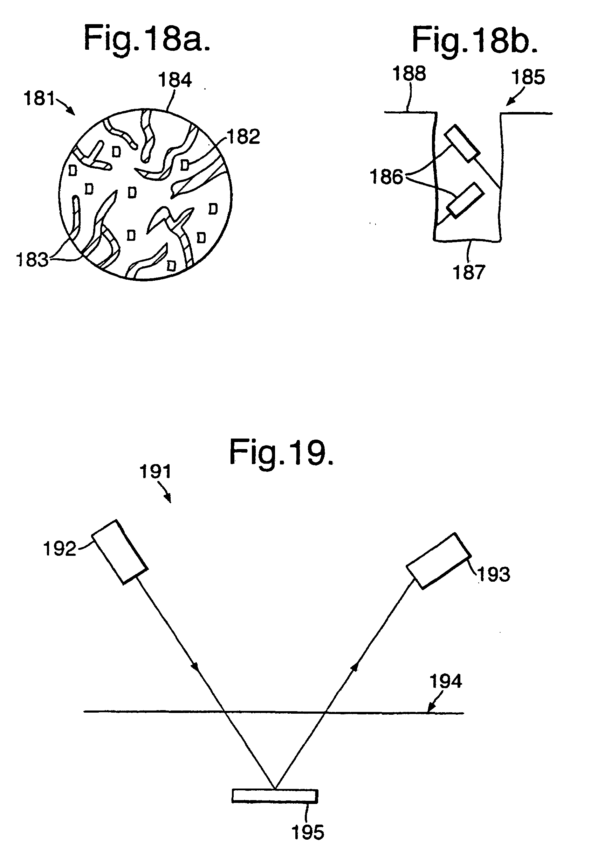

Figure 18a shows a schematic diagram of a radiotherapy device, generally indicated by 181, according to the invention. The radiotherapy device 181 comprises derivatized porous silicon combined with a radio isotope 182 such as $^{90}$Y. The device is in the form of a pellet that may be implanted into an organ in the region of a tumour.

The pellets may be fabricated from a silicon on oxide wafer by a multi-step process. The first step is the formation, by lithographically etching the bulk silicon layer, of a multiplicity of silicon particles bonded to the underlying silicon oxide. The silicon particles are then porosified in an HF solution, the silicon oxide layer being protected with a mask during porosification. Doping with the radioisotope 182 is achieved by immersion of the porosified particles in an aqueous solution of the isotope 182 followed by evaporation. The porous silicon, which now has the isotope 182 located within its pores 183, is annealed to drive the radioisotope 182 into the skeleton 184. The anneal temperature is between 300 C and 1150 C for a period of 30s to 5h. Derivatization of the doped porous silicon is followed by removal from the oxide substrate.

The use of porous silicon allows doping of the pellet throughout its volume. The presence of the radioisotope 182 within the skeleton 184 of the pellet reduces leakage of the isotope 182 to parts of the body other than those being treated. Were the pellets formed from bulk crystalline silicon, this would necessitate doping by ion implantation; a relatively expensive technique that limits the doping depth. Pellets formed from bulk silicon would therefore result in an increased risk of such leakage. The use of derivatized porous silicon means that the corrosion rate, and hence loss of the radioisotope 182, is reduced.

Figure 18b shows a schematic diagram of part of a drug delivery device, generally indicated by 185, according to the invention. The device 185 comprises a sample of derivatized porous silicon in which molecules of a pharmaceutical compound 186 are distributed in the pores 187. The porous silicon is derivatized in such a manner that the pharmaceutical is bonded to the silicon skeleton 188. Derivatization in this way potentially allows a constant rate of release for the pharmaceutical molecules 186 to be achieved.

Figure 19 shows a corrosion analysis system according to the invention, generally indicated by 191. The system 191 comprises a source of electromagnetic radiation 192, a radiation detector 193, and an optical device comprising derivatized porous silicon 195. The device 191 operates by illuminating the mirror 195. Radiation is then reflected by the mirror 195 and detected by the detector 193. The mirror is located within the body 195 of a human or animal patient. As the mirror corrodes in the body 194, its optical properties change and this change may be detected by the detector 193. In this way corrosion of the mirror 195 may be monitored in the body 194.

**Claims**

1. Derivatized porous silicon, having a substantially monomolecular layer, the monomolecular layer comprising one or more organic groups that are covalently bonded by hydrosilylation to at least part of the surface of the porous silicon, the structure and composition of the derivatized porous silicon being such that it is suitable for use as a biomaterial, the derivatized porous silicon being for use as a biomaterial, a biomaterial being a non-living material for use in or on the surface of a living human or animal body.

2. Derivatised porous silicon according to claim 1 **characterised in that** the derivatized porous silicon is derivatized mesoporous silicon.

3. Derivatised porous silicon according to claim 2 **characterised in that** the derivatised porous silicon has a composition and structure such that the corrosion rate of the derivatized mesoporous silicon material in (SHP) is a factor of at least two orders of magnitude lower than underivatized mesoporous silicon.

4. Derivatised porous silicon according to claim 1 **characterised in that** the porosity of the derivatized porous silicon is at least 5%.

5. A biofiltration device, which is adapted for operation in or on the surface of a human or animal body, comprising one or more derivatised porous silicon filters, the or each derivatised porous silicon filter comprising derivatized

13

porous silicon as claimed in claim 1.

6. An immunoisolation device, which is adapted for operation in or on the surface of a human or animal body, comprising a biofiltration device as claimed in claim 5, **characterised in that** the biofiltration device is constructed in such a manner that, when the immunosiolation device is in operation in or on the surface of a human or animal body, at least one of the derivatised porous silicon filters excludes at least some molecules of the immune system from the immunoisolation device.

7. A battery device, which is adapted for operation in or on the surface of a human or animal body, comprising a power source and a biofiltration device as claimed in claim 5.

8. An optical device, which is adapted for operation in or on the surface of a human or animal body, comprising a multilayer mirror comprises a stack of alternating layers of derivatized porous silicon, as claimed in claim 1, having a first porosity and a first refractive index, and derivatized porous silicon having a second porosity and a second refractive index which is higher than the first refractive index.

9. A radiotherapy device, which is adapted for operation in or on the surface of a human or animal body, comprising a pellet formed, at least partly, from a radioisotope and derivatized porous silicon as claimed in claim 1.

10. A microelectrode device, which is adapted for operation in or on the surface of a human or animal body, comprising a plurality of electrical connections and a microelectrode comprising derivatised porous silicon as claimed in claim 1.

**Patentansprüche**

1. Derivatisiertes poröses Silicium mit einer im wesentlichen monomolekularen Schicht, wobei die monomolekulare Schicht eine oder mehrere organische Gruppen aufweist, die über Hydrosilylierung an mindestens einen Teil der Oberfläche des porösen Siliciums kovalent gebunden sind und Struktur und Zusammensetzung des derivatisierten porösen Siliciums derart sind, dass es zur Verwendung als Biomaterial geeignet ist, wobei das derivatisierte poröse Silicium als Biomaterial verwendet wird und ein Biomaterial ein lebloses Material zur Verwendung in oder auf der Oberfläche eines lebenden menschlichen oder tierischen Körpers ist.

2. Derivatisiertes poröses Silicium nach Anspruch 1, **dadurch gekennzeichnet, dass** das derivatisierte poröse Silicium derivatisiertes mesoporöses Silicium ist.

3. Derivatisiertes poröses Silicium nach Anspruch 2, **dadurch gekennzeichnet, dass** das derivatisierte poröse Silicium eine Zusammensetzung und Struktur aufweist, derart, dass die Korrosionsgeschwindigkeit des derivatisierten mesoporösen Siliciummaterials in (SHP) um einen Faktor von mindestens zwei Größenordnungen geringer ist als die von underivatisiertem mesoporösem Silicium.

4. Derivatisiertes poröses Silicium nach Anspruch 1, **dadurch gekennzeichnet, dass** die Porosität des derivatisierten porösen Siliciums mindestens 5 % beträgt.

5. Biofiltrationsvorrichtung, die zum Betrieb in oder auf der Oberfläche eines menschlichen oder tierischen Körpers ausgelegt ist, aufweisend ein oder mehrere derivatisierte poröse Siliciumfilter, wobei das oder jedes derivatisierte poröse Siliciumfilter derivatisiertes poröses Silicium nach Anspruch 1 enthält.

6. Immunisoliervorrichtung, die zum Betrieb in oder auf der Oberfläche eines menschlichen oder tierischen Körpers ausgelegt ist, welche eine Biofiltrationsvorrichtung nach Anspruch 5 aufweist, **dadurch gekennzeichnet, dass** die Biofiltrationsvorrichtung so aufgebaut ist, dass bei Betrieb der Immunisoliervorrichtung in oder auf der Oberfläche eines menschlichen oder tierischen Körpers mindestens eines der derivatisierten porösen Siliciumfilter mindestens einige Moleküle des Immunsystems von der Immunisoliervorrichtung ausschließt.

7. Batterievorrichtung, die zum Betrieb in oder auf der Oberfläche eines menschlichen oder tierischen Körpers ausgelegt ist, welche eine Stromquelle und eine Biofiltrationsvorrichtung nach Anspruch 5 aufweist.

8. Optische Vorrichtung, die zum Betrieb in oder auf der Oberfläche eines menschlichen oder tierischen Körpers ausgelegt ist, welche einen mehrschichtigen Spiegel aufweist, der einen Stapel von alternierenden Schichten von derivatisiertem porösem Silicium nach Anspruch 1 mit einer ersten Porosität und einem ersten Brechungsindex und derivatisiertem porösem Silicium mit einer zweiten Porosität und einem zweiten Brechungsindex, der höher ist als der erste Brechungsindex, umfasst.

9. Radiotherapievorrichtung, die zum Betrieb in oder auf der Oberfläche eines menschlichen oder tierischen Körpers ausgelegt ist, welche ein Pellet aufweist, das mindestens teilweise aus einem Radioisotop und derivatisiertem porösem Silicium nach Anspruch 1 gebildet ist.

10. Mikroelektrodenvorrichtung, die zum Betrieb in oder auf der Oberfläche eines menschlichen oder tierischen Körpers ausgelegt ist, welche eine Vielzahl von elektrischen Anschlüssen und eine Mikroelektrode, die derivatisiertes poröses Silicium nach Anspruch 1 aufweist, umfasst.

**Revendications**

1. Dérivé de silicium poreux, ayant une couche sensiblement monomoléculaire, la couche monomoléculaire comprenant un ou plusieurs groupes organiques qui sont liés de manière covalente par hydrosilylation à au moins une partie de la surface du silicium poreux, la structure et la composition du dérivé de silicium poreux étant telles qu'il est approprié à l'utilisation comme biomatière, le dérivé de silicium poreux étant destiné à l'utilisation comme biomatière, une biomatière étant une matière non vivante destinée à être utilisée dans ou sur une surface d'un corps humain ou animal vivant.

2. Dérivé de silicium poreux selon la revendication 1, **caractérisé en ce que** le dérivé de silicium poreux est un dérivé de silicium mésoporeux.

3. Dérivé de silicium poreux selon la revendication 2, **caractérisé en ce que** le dérivé de silicium poreux possède une composition et une structure telles que le taux de corrosion de la matière du dérivé de silicium mésoporeux en (SHP) est un facteur inférieur d'au moins deux décimales à celui du silicium mésoporeux non dérivé.

4. Dérivé de silicium poreux selon la revendication 1, **caractérisé en ce que** la porosité du dérivé de silicium poreux est au moins de 5 %.

5. Dispositif de biofiltration, qui est adapté au fonctionnement dans ou sur la surface d'un corps humain ou animal, comprenant un ou plusieurs filtres en dérivé de silicium poreux, le ou chacun des filtres en dérivé de silicium poreux comprenant le dérivé de silicium poreux selon la revendication 1.

6. Dispositif d'immunoisolation, qui est adapté au fonctionnement dans ou sur la surface d'un corps humain ou animal, comprenant un dispositif de biofiltration selon la revendication 5, **caractérisé en ce que** le dispositif de biofiltration est construit de telle sorte que, quand le dispositif d'immunoisolation fonctionne dans ou sur la surface d'un corps humain ou animal, au moins l'un des filtres en dérivé de silicium poreux exclut du dispositif d'immunoisolation au moins certaines molécules du système immunitaire.

7. Système de batterie, qui est adapté au fonctionnement dans ou sur la surface d'un corps humain ou animal, comprenant une source d'alimentation et un dispositif de biofiltration selon la revendication 5.

8. Dispositif optique qui est adapté au fonctionnement dans ou sur la surface d'un corps humain ou animal, comprenant un miroir multicouche, qui comprend une succession de couches en alternance de dérivé de silicium poreux selon la revendication 1, ayant une première porosité et un premier indice de réfraction, et de dérivé de silicium poreux ayant une seconde porosité et un second indice de réfraction qui est supérieur au premier indice de réfraction.

9. Dispositif de radiothérapie, qui est adapté au fonctionnement dans ou sur la surface d'un corps humain ou animal, comprenant une pastille formée au moins partiellement à partir d'un radioisotope et d'un dérivé de silicium poreux selon la revendication 1.

10. Dispositif à microélectrode, qui est adapté au fonctionnement dans ou sur la surface d'un corps humain ou animal, comprenant une pluralité de connexions électriques et une microélectrode comprenant du dérivé de silicium poreux selon la revendication 1.

# Fig.1.

## Fig.2a.

## Fig.2b.

## Fig.2c.

## Fig.2d.

Fig.3a.

Fig.3b.

Fig.3c.

# Fig.4.

# Fig.5a.

# Fig.5b.

# Fig.5c.

# Fig.6a.

# Fig.6b.

# Fig.7.

# Fig.8.

# Fig.9.

# Fig.10a.

# Fig.10b.

Fig.11a.

Fig.11b.

Fig.11c.

Fig.12a.

Fig.12b.

Fig.12c.

Fig.13a.

Fig.13b.

EP 1 175 233 B1

# Fig.14.

# Fig.15.

# Fig.16.

# Fig.17a.

# Fig.17b.

# Fig.18a.

181 184 182 183

# Fig.18b.

188 185 186 187

# Fig.19.

191 192 193 194 195